# EUROPÄISCHE PATENTSCHRIFT

(11) **EP 0 795 535 B1**
(45) Veröffentlichungstag und Bekanntmachung des Hinweises auf die Patenterteilung: **05.09.2001**
(21) Anmeldenummer: 97101728.0
(22) Anmeldetag: 04.02.1997
(51) Int. Cl.: C07C 67/08, C07C 67/54, C07C 69/54

(54) **Verfahren und Vorrichtung zur kontinuierlichen Herstellung von Alkylestern der (Meth)acrylsäure**
Process and equipment for the continuous preparation of alkyl esters of (meth)acrylic acid
Procédé et installation pour la fabrication continue d'esters alkyliques d'acide (méth)acrylique

(30) Priorität: 06.02.1996 DE 19604252
(43) Veröffentlichungstag der Anmeldung: 17.09.1997
(73) Patentinhaber: BASF AKTIENGESELLSCHAFT, 67056 Ludwigshafen (DE)
(72) Erfinder: Dams, Albrecht, Dr., 67157 Wachenheim (DE); Herbst, Holger, Dr., 67227 Frankenthal (DE); Aichinger, Heinrich, Dr., 68199 Mannheim (DE); Nestler, Gerhard, Dr., 67061 Ludwigshafen (DE); Exner, Herbert, Dr., 67165 Waldsee (DE); Iffland, Gabriele, (FH), D.I., 67071 Ludwigshafen (DE); Weck, Alexander, (FH), D.I., 67251 Freinsheim (DE)
(74) Vertreter: Isenbruck, Günter, Dr.

(56) Entgegenhaltungen:
- EP-A- 0 694 524
- DE-A- 2 552 987

## Beschreibung

Die Erfindung betrifft ein Verfahren und eine Vorrichtung zur kontinuierlichen Herstellung von Alkylestern der (Meth)acrylsäure durch Umsetzung von (Meth)acrylsäure mit 4 bis 5 C-Atome aufweisenden Alkanolen.

Der Begriff (Meth)acrylsäure bezeichnet in bekannter Weise Acryl- oder Methacrylsäure. Alkylester der (Meth)acrylsäure sind allgemein bekannt und z.B. als Ausgangsmonomere zur Herstellung wäßriger Polymerdispersionen von Bedeutung, die z.B. als Klebstoffe Verwendung finden.

Verfahren zur Herstellung von (Meth)acrylsäurealkylestern durch Umsetzung von (Meth)acrylsäure mit 1 bis 5 C-Atomen aufweisenden einwertigen Alkanolen in homogener flüssiger Phase, bei erhöhter Temperatur und in Gegenwart Protonen liefernder Katalysatoren sind bekannt und z.B. in den DE-A 14 68 932, 22 26 829 und 22 52 334 beschrieben. Es handelt sich hierbei um typische Gleichgewichtsreaktionen, bei denen der Umsetzungsgrad der (Meth)acrylsäure und des jeweiligen Alkanols zum entsprechenden Ester durch die Gleichgewichtskonstante signifikant begrenzt ist. Dies hat zur Folge, daß für eine wirtschaftliche Verfahrensführung die nicht umgesetzten Ausgangsstoffe vom gebildeten Ester abgetrennt und in die Reaktionszone zurückgeführt werden müssen. Als besonders schwierig erweist sich dabei in der Regel die Abtrennung des gebildeten Esters von nicht umgesetzter (Meth)acrylsäure, da deren Siedepunkte meist vergleichsweise eng zusammenliegen. Es wurden daher bereits verschiedene Maßnahmen zur Erhöhung des Umsatzes der (Meth)acrylsäure zu den entsprechenden Estern vorgeschlagen, wie die Anwendung eines erhöhten molaren Überschusses an Alkanol gegenüber der (Meth)acrylsäure, die Entfernung des Reaktionswassers mittels eines ein geeignetes Azeotrop bildenden organischen Schleppmittels oder die Extraktion des gebildeten Esters mit einem geeigneten Lösungsmittel während der Reaktion. Diese Verfahren weisen jedoch den Nachteil auf, daß ein großer Überschuß an Alkanol zurückgewonnen bzw. das Schleppmittel oder das Extraktionsmittel isoliert werden müssen. Ein erhöhter Alkanolüberschuß verstärkt zudem die Bildung von dessen Dialkylether als Nebenprodukt.

Die GB-B 1 017 522 offenbart ein Verfahren zur Herstellung von n-Butylacrylat. Als Veresterungsbedingungen empfiehlt sie dabei ein molares Verhältnis von (Ausgangs)alkanol zu (Ausgangs)säure von 2,3 bis 5, sowie einen auf die Gesamtmasse der Reaktanden bezogenen Gehalt an katalytisch wirksamer Schwefelsäure oder organischer Sulfonsäure von 0,5 bis 5 Gew.-%. Nachteilig an dieser Verfahrensweise ist der erforderliche erhöhte Überschuß an Alkanol, der die Bildung an unerwünschtem Dialkylether fördert, sowie die unter vorgenannten Bedingungen nicht voll befriedigende Ausbeute an n-Butylacrylat bezogen auf die eingesetzte Menge an Acrylsäure.

Aus der DE-C 25 52 987 ist ein Verfahren zu kontinuierlichen Herstellung von Alkylestern der Acrylsäure durch Umsetzung von Acrylsäure und 1 bis 4 C-Atome aufweisenden einwertigen Alkanolen in homogener, flüssiger, an Lösungsmittel freier Phase im Molverhältnis von 1(Alkanol):1(Acrylsäure) bis 2(Alkanol):1(Acrylsäure) bei erhöhter Temperatur und in Gegenwart von Schwefelsäure oder organischer Sulfonsäure als Katalysator bekannt, bei dem man die Acrylsäure, das Alkanol und den Katalysator kontinuierlich einer Reaktionszone zuführt, während einer Verweilzeit von einigen Stunden den gebildeten Acrylsäurealkylester als Bestandteil wenigstens eines neben dem Acrylsäurealkylester als weiteren Bestandteil aus Wasser oder Wasser und Alkanol bestehenden wäßrigen Azeotrops über Kopf einer der Reaktionszone aufgesetzten, einen Kopfdruck von 0,1 bis 1 atm aufweisenden, Rektifikationskolonne rektifikativ abtrennt, das anfallende Destillat in eine den gebildeten Acrylsäureester enthaltende organische und in eine wäßrige Phase auftrennt, einen Teil der organischen Phase zum Zwecke der Erzeugung einer erhöhten Trennwirkung sowie gegebenenfalls einen Teil der wäßrigen Phase zur Aufrechterhaltung der Zusammensetzung des wäßrigen Azeotrops über Kopf der Rektifikationszone zurückführt, aus der überschüssigen organischen Phase den Alkylester in an sich bekannter Weise abtrennt und einen Teil des Reaktionsgemisches aus der Reaktionszone austrägt, von Hochsiedern destillativ befreit und das dabei anfallende Destillat in die Reaktionszone zurückführt.

Vorrangige Zielsetzung der DE-C 25 52 987 ist die Vermeidung unerwünschter Etherbildung aus Alkanol. Nachteilig an der Verfahrensweise der DE-C 25 52 987 ist jedoch, daß trotz destillativer Behandlung des Austrags aus dem Reaktionsgemisch und Rückführung des dabei anfallenden Destillats in die Reaktionszone die Ausbeute an Alkylacrylat, bezogen auf eingesetzte Acrylsäure, nicht zu befriedigen vermag. Auch die erreichte Verringerung der Dialkylether-Nebenproduktbildung kann nicht voll befriedigen. Ferner vermag auch die gemäß der Ausführungsbeispiele erforderliche Verweilzeit nicht zu befriedigen. Dies gilt auch für die Raum-Zeit-Ausbeute. Es wird angenommen, daß dies auf der geringen Konzentration an Katalysator beruht.

Es ist daher in der prioritätsälteren, nicht-vorveröffentlichten EP-A 0 733 617 vorgeschlagen worden, das entsprechende Veresterungsverfahren im Beisein erhöhter Konzentrationen an Katalysator durchzuführen, was die Rückspaltung von bei der Veresterung als weitere Nebenprodukte gebildeten Oxyestern fördert, und damit bei gegebener Verweilzeit die auf eingesetzte (Meth)acrylsäure bezogene Ausbeute an Ester erhöht.

Weiterhin ist in der prioritätsälteren, nicht-vorveröffentlichten EP-A-765 859 bereits vorgeschlagen worden (europäische Patentanmeldung 96 11 5454.9 vom 26.9.1996), bei gleichzeitig hoher Ausbeute an Ester eine weitere Absenkung der Dialkylethermenge dadurch zu erreichen, daß die Reaktionszone aus einer Kaskade von mindestens zwei hintereinander geschalteten, vorzugsweise kontinuierlich betriebenen, Reaktionsbereichen besteht, und der flüssige Austragstrom eines Reaktionsbereichs den Zulaufstrom des nachfolgenden Reaktionsbereichs bildet.

Der Erfindung liegt die Aufgabe zugrunde, ein Veresterungsverfahren zur Herstellung von Alkylestern der (Meth)acrylsäure zu schaffen, das neben einer optimierten Ausbeute mildere Reaktionsbedingungen und damit neben stark verminderter Etherbildung weniger Hochsiederbildung, eine hohe Raum-Zeit-Ausbeute, eine erhöhte Flexibilität des Betriebs der Anlage sowie niedrige Investitionskosten aufgrund einer minimierten Apparatezahl ermöglicht.

Die die Lösung der gestellten Aufgabe darstellende Erfindung geht aus von dem bekannten Verfahren zur kontinuierlichen Herstellung von Alkylestern der (Meth)acrylsäure durch Umsetzung von (Meth)acrylsäure mit 4 bis 5 C-Atome aufweisenden Alkanolen in homogener, flüssiger, an Lösungsmittel freier Phase bei erhöhter Temperatur und in Gegenwart eines sauren Veresterungskatalysators, bei dem man die (Meth)acrylsäure, das Alkanol und den Katalysator einer Reaktionszone zuführt, während einer Verweilzeit das gebildete Wasser als Bestandteil eines Alkanol umfassenden Gemisches in einer der Reaktionszone aufgesetzten Rektifikationseinheit rektifikativ abtrennt, das dabei anfallende Destillat in eine Alkanol enthaltende organische und in eine Wasser enthaltende wäßrige Phase auftrennt, die organische Phase in die Rektifikationseinheit zurückführt, das Reaktionsgemisch aus der Reaktionszone austrägt und in eine weitere Rektifikationseinheiten umfassende destillative Trennzone leitet und in dieser den gebildeten Alkylester der (Meth)acrylsäure abtrennt.

Das erfindungsgemäße Verfahren ist dann dadurch gekennzeichnet, daß
a) (Meth)acrylsäure und Alkanol im Molverhältnis 1:0,75 bis 1:2 umgesetzt werden,
b) die in der Rektifikationseinheit III anfallende organische Phase vollständig oder mit Ausnahme eines Teilstroms, der ausgeschleust wird, in die Rektifikationseinheit zurückgeführt wird,
c) die in der Rektifikationseinheit III anfallende wäßrige Phase ausgeschleust wird,
d) das aus der Reaktionszone ausgetragene Reaktionsgemisch unter Zusatz von Wasser einer weiteren Rektifikationseinheit I zugeführt und in dieser in ein den Katalysator und die verbliebene (Meth)acrylsäure enthaltendes Produkt II und in ein den Alkylester der (Meth)acrylsäure, verbliebenes Alkanol und Wasser enthaltendes Produkt I aufgetrennt wird,
e) das in der Rektifikationseinheit I anfallende Produkt II mit Ausnahme eines Teilstroms, der ausgeschleust wird, in die Reaktionszone zurückgeführt wird,
f) das Produkt I der Rektifikationseinheit I in eine den Alkylester der (Meth)acrylsäure enthaltende organische Phase und in eine wäßrige Phase aufgetrennt und
g) die in der Rektifikationseinheit I anfallende organische Phase einer weiteren Rektifikationseinheit II zugeführt und in dieser der Alkylester der (Meth)acrylsäure vom verbliebenen Alkanol abgetrennt und das verbliebene Alkanol in die Reaktionszone zurückgeführt wird.

Der Begriff Rektifikationseinheit ist hier wie auch im folgenden als allgemeine Bezeichnung für Apparate zu verstehen, in denen durch Wärmezufuhr Dämpfe erzeugt werden, die aufsteigen und in Kontakt mit abströmender flüssiger Phase stehen. Hierzu sind auch einfache Destillationskolonnen zu rechnen. In der Regel handelt es sich hierbei jedoch um Rektifikationskolonnen, in denen Einbauten für den intensiven Kontakt zwischen Flüssigkeit und Dampf enthalten sind. Derartige Einbauten sind Böden, wie Glockenböden, Lochböden, insbesondere Dual-Flow-Böden, Schüttungen, Packungen oder dergleichen. Zur Vereinfachung des Verständnisses der Zusammenhänge sind die verschiedenen Rektifikationseinheiten mit römischen Ziffern bezeichnet. Auch die verschiedenen, im einzelnen beschriebenen Produkte tragen eine derartige Bezeichnung.

Aufgrund der relativen Schwerflüchtigkeit des sauren Veresterungskatalysators, sowie aufgrund der Abtrennung des Wassers aus der Reaktionszone über die Rektifikationseinheit III, nimmt der auf die in der jeweiligen in der Veresterung wie auch in der Rektifikationseinheit I enthaltende Menge an Reaktionsgemisch bezogene Gewichtsanteil an Katalysator innerhalb der Reaktionszone von der ersten Reaktionszone zur zweiten Rektifikationszone - bei Vorhandensein mehrerer Reaktionsbereiche - auch von Reaktionsbereich zu Reaktionsbereich zu.

Die Veresterung wird bei vermindertem Druck zur Entfernung des Reaktionswasser betrieben und ist von der ihr nachfolgenden Abtrennung des (Meth)acrylsäurealkylesters räumlich und auch regeltechnisch getrennt. Veresterung und nachfolgende Abtrennung des (Meth)acrylsäurealkylesters in der Rektifikationszone sind daher sehr flexibel einstellbar. Wasser, das zur azeotropen Entfernung des (Meth)acrylsäurealkylesters in die zweite Rektifikationszone geleitet wird, beeinflußt daher die Veresterung nur geringfügig.

Die Reaktionszone besteht aus einem oder mehreren Reaktionsbereichen. Bei der Ausführungsform der Erfindung mit mehreren Reaktionsbereichen ist es vorteihaft, diese zu kaskadieren. Der flüssige Austragsstrom eines Reaktionsbereiches bildet dabei den Zulauf des nachfolgenden Reaktionsbereiches. Dies kann mit Hilfe eines Überlaufes geschehen. Für den Fall, daß es sich bei den einzelnen Reaktionsbereichen um voneinander getrennte Apparate handelt, ist deren Anzahl unter Berücksichtigung der Investitionskosten ≥2 und ≤4. Wird mehr als ein Reaktionsbereich innerhalb ein und desselben Reaktors geschaffen (z.B. durch den Einsatz von Trennblechen), so kann die Anzahl der Reaktionsbereiche auch größer 4 sein. Im Falle von mehreren Reaktionsbereichen werden die Brüden der Reaktionsbereiche einer gemeinsamen Rektifikationskolonne zugeführt, deren flüssiger Ablauf vorteilhafterweise in den ersten Reaktionsbereich gelangt. Das Destillat wird nach der Kondensation in zwei Phasen, eine organische, weitgehend aus Alkanol bestehend, und eine wäßrige Phase, weitgehend aus Wasser bestehend, geteilt und die organische Phase wird im wesentlichen vollständig, bevorzugt vollständig, zurück zur Rektifikationseinheit III geleitet.

Die Temperatur des Reaktionsgemisches in den verschiedenen Reaktionsbereichen entspricht normalerweise der dem eingestellten Druck, vorzugsweise 0,1 bis 1 atm, besonders bevorzugt 0,1 bis 0,5 atm, entsprechenden Siedetemperatur des jeweiligen Reaktionsgemisches. D.h. sie nimmt normalerweise längs der Kaskade (im Falle von mehreren Reaktionsbereichen) bis hin zum Sumpf der Rektifikationseinheit I zu.

Die Trennung von Veresterungsreaktion und destillativer Abtrennung des Alkylesters der (Meth)acrylsäure erlaubt mildere Reaktionsbedingungen. Die Reaktion kann in allen Reaktionsbereichen bei einem Druck von 100 mbar bis Atmosphärendruck, vorzugsweise bei 200 bis 700, besonders bevorzugt bei 300 bis 450 mbar Kopfdruck (Wasserabtrennkolonne) und einer Temperatur *im ersten Reaktionsbereich von 70 bis 150°C, vorzugsweise von 80 bis 130°C und im letzten Bereich von 100 bis 160°C, vorzugsweise von 105 bis 130°C* betrieben werden. Der Druck kann dabei in allen Reaktionsbereichen gleich sein. Die Rektifikationseinheit I wird bevorzugt bei Normaldruck und bei ≥ 100°C und ≤ 130°C betrieben. Die Temperatur in der der Reaktionszone nachgeschalteten Rektifikationseinheit I sollte 135°C nicht überschreiten, um unerwünschte Polymerisationen als Nebenreaktion zu unterdrüken.

Gemäß einer vorteilhaften Ausbildung der Erfindung beträgt der Gehalt an katalytisch wirksamer Säure im ersten Reaktionsbereich, bezogen auf das darin enthaltene Reaktionsgemisch, 0,1 bis 10 Gew.-%, bevorzugt zwischen 0,1 und 6 Gew.-% para-Toluolsulfonsäure oder einer dazu äquimolaren Menge an einer anderen organischen Sulfonsäure und/oder Schwefelsäure. Der entsprechende Säuregehalt im Sumpf der Rektifikationseinheit I, bezogen auf das darin enthaltene Gemisch, beträgt vorzugsweise 2,5 bis 25 Gew.-%. Die Gesamtverweilzeit der Reaktanden in der Reaktionszone beträgt in der Regel 0,25 bis 15 Stunden, vorzugsweise 1 bis 7 h, besonders bevorzugt 2 bis 5 h. In aufeinander folgenden Bereichen nimmt sie vorzugsweise ab. Im Sumpf der Rektifikationseinheit I beträgt sie vorzugsweise 0,2 bis 5 Stunden. In der Rektifikationseinheit I erfolgt aufgrund des erhöhten Katalysatorgehalts eine Teilspaltung der in der Veresterung geringfügig gebildeten Oxyester (in der Hauptsache Alkoxyester und (Meth)acryloxyester der (Meth)acrylsäure). Dies ist ein wichtiger Vorteil des erfindungsgemäßen Verfahrens.

Ein Teil der Flüssigkeit wird kontinuierlich aus der ersten, der Reaktionszone nachgeschalteten Rektifikationseinheit I in die Reaktionszone, vorzugsweise in den ersten Reaktionsbereich, zurückgeführt. Ein weiterer Teil der Sumpfflüssigkeit dieser Rektifikationseinheit I wird zur Ausschleusung von Hochsiedern, vorzugsweise kontinuierlich, einer weiteren Destillationseinheit zugeführt, in der die Leichtsieder von den Hochsiedern (gebildete Oligomere und Polymere), vorzugsweise einstufig und diskontinuierlich, abgetrennt werden. Diese Leichtsieder stellen im wesentlichen Alkyl(meth)acrylat, Wasser, Alkanol und (Meth)acrylsäure dar. Sie werden zur Ausbeuteerhöhung der Rektifikationseinheit I zugeführt. In der zusätzlichen Destillationseinheit wird ebenfalls ein Teil der Oxyester gespalten, so daß die Verluste an Wertstoffen sehr niedrig gehalten werden können.

Mit Vorteil wird aus der Rektifikationseinheit I eine Teilmenge, die zwischen 20 Gew.-% und 95 Gew.-%, vorzugsweise 35 bis 55 Gew.-% der dieser Rektifikationseinheit I aus dem letzten Reaktionsbereich zugeführten Menge entspricht, in die Reaktionszone zurückgeführt. Um den Anteil an hochsiedenden, nicht spaltbaren Nebenprodukten zu begrenzen, genügt es, aus der Rektifikationseinheit I eine Menge von 1 bis 20 Gew.%, bevorzugt 2 bis 10 Gew.-%, bezogen auf die Zulaufmenge an Edukten zur Reaktionszone, in eine weitere Destillations-(Rektifikations)einheit IV auszuschleusen. Die Menge an aus dieser Destillations-(Rektifikations)einheit IV ausgeschleusten Hochsiedern beträgt 3 bis 30 Gew.-%, in der Regel 5-15 Gew.-% bezogen auf die dieser Zone zugeführte Menge. Die Gesamtverluste, bezogen auf gebildetes Alkylacrylat, belaufen sich auf weniger als 1,5 %.

Entsprechend der aus der Destillations-(Rektifikations)einheit IV in der ausgeschleusten Hochsiedermenge enthaltenden Katalysatormenge wird in den ersten Reaktionsbereich, vorzugsweise kontinuierlich, Frisch-Katalysator ergänzt. Dies führt zu stationären Zuständen der erforderlichen Konzentration an Katalysator in der Reaktionszone und in der Rektifikationseinheit I.

Durch die Kreisführung wird eine Katalysatoraufbereitung überflüssig und der Bedarf an frischem Katalysator wird reduziert. Bei der Ausschleusung geht auch Prozeßstabilisator mit, so daß sich dessen Gehalt auf einen stationären Wert einpendelt.

Die auf die Menge der eingesetzten (Meth)acrylsäure bezogenen Umsätze liegen typischerweise bei ≥ 95 mol.-%. Mit Vorteil wird im ersten Reaktionsbereich bereits bei einem ≥ 90 mol.-% liegenden Umsatz gefahren. Bei Verwendung mehrerer hintereinandergeschalteter Reaktionsbereiche (Kaskade) sind diese vorteilhafterweise so zu gestalten, daß die Verweilzeit längs der Kaskade von Reaktionsbereich zu Reaktionsbereich abnimmt.

Das molare Einsatzverhältnis von (Meth)acrylsäure zu Alkanol beträgt 1:0,75 bis 1:2. Von besonderem Vorteil ist ein stöchiometrisches Verhältnis von 1:1. Ein stöchiometrischer Einsatz der Edukte hat den Vorteil, daß neben der niedrigen Temperatur in der Veresterung eine sehr starke Absenkung der Dialkyletherbildung erreicht wird. Weiterhin hat dies zur Folge, daß im Sumpf der Rektifikationseinheit I bei erhöhten Katalysatorkonzentrationen ein Überschuß an (Meth)acrylsäure besteht, der wiederum den Vorteil besitzt, daß das aus der gleichzeitig erfolgenden in-situ-Spaltung entstehende Alkanol zu (Meth)acrylsäurealkylester umgesetzt wird, anstatt zu Dialkylether weiterzureagieren.

Besonders vorteilhaft ist es, als Veresterungskatalysatoren para-Toluolsulfonsäure und/oder andere organische Sulfonsäuren und/oder Schwefelsäure einzusetzen. Der Gehalt an katalytisch wirksamer Säure in der Reaktionszone, bezogen auf das darin enthaltene Reaktionsgemisch, kann 0,1 bis 6 Gew.-% para-Toluolsulfonsäure und/oder einer dazu äquimolaren Menge an weiteren organischen Sulfonsäuren und/oder Schwefelsäure betragen. Der Gehalt an katalytisch wirksamer Säure im Sumpf der Rektifikationseinheit I, bezogen auf das darin enthaltene Reaktionsgemisch, kann zwischen 2,5 und 25 Gew.-% para-Toluolsulfonsäure und/oder einer dazu äquimolaren Menge an einer anderen organischen Sulfonsäure und/oder Schwefelsäure betragen.

Da die eingesetzte Acrylsäure geringe Anteile an Essigsäure enthält, fallen neben Dialkylether als Nebenprodukte Alkylester der Essigsäure an. Beide Nebenkomponenten gehen über Kopf der der Reaktionszone aufgesetzten Rektifikationseinheit III mit und verbleiben bei der destillativen Wasserabtrennung im Alkanol, also der organischen Phase der Rektifikationseinheit III und werden zweckmäßigerweise in die Reaktionszone zurückgeführt. Dadurch reichern sich beide Verunreinigungen in der organischen Phase an. Besonders vorteilhaft ist es, eine an Essigsäure verarmte Roh-Acrylsäure einzusetzen. In diesem Fall genügt es, aufgrund der sehr geringen Mengen an Dialkylether und Alkylacetat, einen Teilstrom der organischen Phase auszuschleusen, in der Regel weniger als 1 % der eingesetzten Alkanolmenge. Auf diese Weise werden Leichtsieder aus dem System ausgeschleust und gelangen nicht ins Reinprodukt.

Eine weitere Ausführungsform besteht darin, daß ein Teilstrom der organischen Phase des Kopfprodukts der der Reaktionszone aufgesetzten Rektifikationskolonne III in einer weiteren Kolonne in ein Kopfprodukt, bestehend aus Dialkylether, Alkylacetat, Alkanol und Wasser, und ein Sumpfprodukt, weitgehend bestehend aus Alkanol aufgetrennt wird. Das Alkanol aus dem Sumpf dieser Rektifikationskolonne für die Leichtsieder-Abtrennung wird in der vorher beschriebenen Weise in die Reaktionszone, bevorzugt über die Rektifikationskolonne III, zurückgeführt.

Die organische Phase des Kopfprodukts der Rektifikationseinheit I enthält den (Meth)acrylsäurealkylester als Hauptkomponente neben Alkanol und Wasser. (Meth)acrylsäure und Alkoxialkylester der (Meth)acrylsäure (im weiteren auch kurz Alkoxiester genannt) gelangen durch geeignete Einstellung der Betriebsparameter in dieser Rektifikationskolonne nicht in das Kopfprodukt und bedürfen keiner weiteren Abtrennung.

Die nachgeschaltete Rektifikationseinheit II (Alkanol/Ester-Abtrennung) wird bevorzugt derart betrieben, daß am oberen Ende dieser Rektifikationskolonne II als Kopfprodukt Alkanol mit geringen Anteilen Wasser und Alkylacrylat entnommen wird, das zurück in die Reaktionszone geleitet wird, und daß am unteren Ende der Rektifikationskolonne reines Alkyl(meth)acrylat entnommen wird. Eine besonders bevorzugte Ausführungsform der Alkanol/-Ester-Abtrennung besteht darin, daß der Reinester am unteren Ende der Rektifikationskolonne II oberhalb des Verdampfers, zwischen Verdampfer und fünftem Boden, am geeignesten oberhalb des Verdampfers als dampfförmiger Seitenabzug entnommen wird. Dieser Gasstrom wird kondensiert und in bekannter Weise mit Lagerstabilisator (z.B. Hydrochinonmonomethylether), umstabilisiert. Dem Verdampfer der Rektifikationskolonne II, die der Alkanol/Ester-Abtrennung dient, wird zur Vermeidung der Bildung von Hochsiedern kontinuierlich ein Teilstrom, in der Regel zwischen 1 bis 20 %, bevorzugt 1 bis 5 % der Zulaufmenge dieser Kolonne, entnommen und zur Reaktionszone oder bevorzugt zur Rektifikationseinheit I zurückgeführt. Eine weitere Reinigung des dampfförmig entnommenen Reinesters ist nicht erforderlich.

Eine günstige Ausführungsform besteht darin, daß das Kopfprodukt der Rektifikationskolonne II für die Alkanol/Ester-Trennung in den oberen Teil der, der Reaktionszone aufgesetzten Rektifikationskolonne III zurückgeführt wird, um darin enthaltenes Wasser nicht in die Reaktionsmischung gelangen zu lassen.

Eine weitere Ausführungsform besteht darin, daß der (Meth)acrylsäurealkylester am Sumpf der Alkanol/Ester-Trennkolonne (Rektifikationskolonne II) flüssig entnommen, und in einer nachgeschalteten Schwersieder-Rektifikationskolonne der gewünschten Rein-(Meth)acrylsäureester über Kopf abgetrennt wird. Die die schwerer siedenden Nebenprodukte enthaltende Sumpfflüssigkeit der Schwersieder-Rektifikationskolonne wird zweckmäßigerweise in die Reaktionszone und/oder zur Rektifikationseinheit I zurückgeführt, vorzugsweise auf direktem Weg.

Besonders bevorzugt wird das erfindungsgemäße Verfahren zur Herstellung von n-Butylacrylat angewendet.

Die in der erfindungsgemäß aus der von der Reaktionszone räumlich getrennten Rektifikationseinheit I entstehenden Dämpfe werden, wie bereits beschrieben, einer Rektifikationszone zugeführt. Hinsichtlich des aus dieser über Kopf abgetrennten, den Zielester enthaltenden, Gemisches lassen sich im wesentlichen zwei Fallgestaltungen unterscheiden. Handelt es sich um ein Heteroazeotrop, wie im Fall der Herstellung von n-Butylacrylat, scheidet sich das Azeotrop nach seiner Kondensation von selbst in eine wäßrige und in eine organische Phase. Die wäßrige Phase besteht normalerweise hauptsächlich aus Wasser und etwas Alkanol, die organische Phase besteht in der Regel im wesentlichen aus dem gebildeten Ester und Alkanol. Zur Einstellung der rektifikativen Trennwirkung führt man einen entsprechenden Teil der organischen Phase über Kopf der Rektifikationszone zurück.

Zur Aufrechterhaltung der Zusammensetzung des wäßrigen Azeotrops führt man einen entsprechenden Teil der wäßrigen Phase in die Rektifikationszone I zurück, vorzugsweise ebenfalls über Kopf der aufgesetzten Rektifikationskolonne. Aus dem nicht zurückgeführten Anteil der wäßrigen Phase läßt sich enthaltenes Alkanol z.B. durch Strippen (z.B. mit Luft oder Wasserdampf) abtrennen und in die Reaktionszone zurückführen. Zweckmäßigerweise erfolgt die Rückführung auf direktem Weg. Das dabei anfallende, im wesentlichen reine Wasser wird ausgetragen.

Handelt es sich bei dem im Rahmen des erfindungsgemäßen Verfahrens kontinuierlich über Kopf einer Rektifikationszone abgetrennten, den Zielester enthaltenden, wäßrigen Azeotrop um kein Heteroazeotrop, so scheidet sich dieses nach seiner Kondensation nicht von selbst in eine wäßrige und in eine organische Phase. Diese Auftrennung kann jedoch in einfacher Weise z.B. dadurch erzielt werden, daß man das im Azeotrop enthaltene Alkanol mittels Wasser extrahiert und das dabei anfallende Wasser/Alkanol-Gemisch rektifikativ auftrennt. Das Alkanol wird zweckmäßigerweise in die Reaktionszone zurückgeführt, vorzugsweise über Kopf der aufgesetzten Rektifikationszone.

Eine besonders bevorzugte Ausführungsform bei Vorliegen eines Heteroazeotrops besteht darin, die aus der am Kopf der Reaktionszone aufgesetzten Rektifikationskolonne III anfallende überschüssige wäßrige Phase (Reaktionswasser aus der Veresterung) zum Kopfprodukt der Rektifikationszone I zu leiten. Die wäßrige Phase dieses Heteroazeotrops nimmt weniger Alkanol nach der Entmischung der Phasen aufgrund des hohen Gehalts an (Meth)-acrylsäurealkylester und des geringeren Alkanolgehalts in der organischen Phase auf. Aus dieser am Kopf der Rektifikationseinheit I erhaltenen Wasserphase kann das überschüssige Reaktionswasser, das zwischen 1 Gew.-% und 5 Gew.-%, im Durchschnitt 2,5 Gew.-% Alkanol enthält, ausgeschleust werden. In der Regel kann eine Strippung des Alkanols als weiterer Verfahrensschritt entfallen.

Üblicherweise enthält das aus der Rektifikationseinheit I abgezogene Azeotrop, bei richtiger Einstellung der rektifikativen Trennwirkung, keine Ausgangssäure. Sollte letzteres jedoch nicht der Fall sein, lassen sich diese extraktiv mittels Wasser oder einer alkalischen Lösung abtrennen und das Extrakt gegebenenfalls anschließend in an sich bekannter Weise aufarbeiten.

Beim erfindungsgemäßen Verfahren erfolgen sowohl die Veresterungsreaktion als auch die thermischen Trennungen vorzugsweise in Gegenwart von üblichen Mengen an sich üblicher Polymerisationsinhibitoren. In der Regel werden, bezogen auf die Menge der α,β-monoethylenisch ungesättigten Monomeren, 0,01 bis 0,1 Gew.-% eines geeigneten Polymerisationsinhibitors eingesetzt. Sie werden mit Vorteil am Kopf der der Reaktionszone aufgesetzten Rektifikationskolonne III, am Kopf der Rektifikationseinheit I und am Kopf der Rektifikationseinheit II (Alkanol/Ester-Trennkolonne) zugegeben. Als Polymerisationsinhibitoren kommen z.B. in Betracht phenolische Verbindungen wie Hydrochinon, Hydrochinonmonomethylether, aber auch p-Benzochinon, Phenothiazin, Methylenblau, Phenylendiamine und/oder Luft.

Das erfindungsgemäße Verfahren zeichnet sich im Vergleich zu den Verfahren des Stands der Technik durch eine deutliche Reduzierung von Teilschritten und Trennoperationen, eine hohe Flexibilität aufgrund der Separierung von Wasserentfernung aus der Veresterung und Abtrennung des (Meth)acrylsäurealkylesters durch Wasserzugabe, durch reduzierte Verweilzeiten, eine erhöhte Ausbeute an gewünschtem Ester bezogen auf eingesetzte Ausgangssäure, eine reduzierte Ethermenge, geringe Hochsiedernebenproduktbildung und damit einen reduzierten Austrag der Flüssigphase aus der Reaktions- und der Rektifikationseinheiten sowie dadurch aus, daß eine Nachspaltung von Hochsiederausträgen und Rückgewinnung von Wertstoffen in einem einfachen Verdampfer/ Kondensatorsystem (vierte Rektifikationseinheit) ohne weitere Trennkolonne geschieht. Die so erzielten geringen Verluste aufgrund von Hochsiederausschleusungen sind auf eine partielle Rückspaltung von relativ hochsiedenden Oxiestern (z.B. Alkoxypropionsäureester) in den Rektifikationseinheiten I und IV zurückzuführen.

Eine günstige Ausführungsform besteht darin, daß das Gesamtverfahren mit insgesamt drei Trennkolonnen-(einheiten) auskommt:
1. einer Rektifikationseinheit III zur Entfernung von Wasser aus der Reaktion,
2. einer Rektifikationseinheit I zur Abtrennung des (Meth)acrylsäurealkylesters von Hochsiedern, Acrylsäure und Katalysator
3. einer Rektifikationseinheit II zur Rückführung des Alkanols mit Gewinnung des Reinesters im Seitenabzug.

Eine andere Ausführungsform bedingt zwei zusätzliche Trennkolonnen zur Entfernung von Acetat aus der organischen Phase des Kopfprodukts der der Reaktionszone aufgesetzten Rektifikationseinheit III sowie zur Strippung von Alkanol aus dem Reaktionswasser. Diese Trennkolonnen(-einheiten) bedingen jedoch aufgrund der geringen abzutrennenden Mengen nur einen geringfügigen Investitionsbetrag und beeinträchtigen in keinerlei Weise die anderen Vorzüge des Verfahrens.

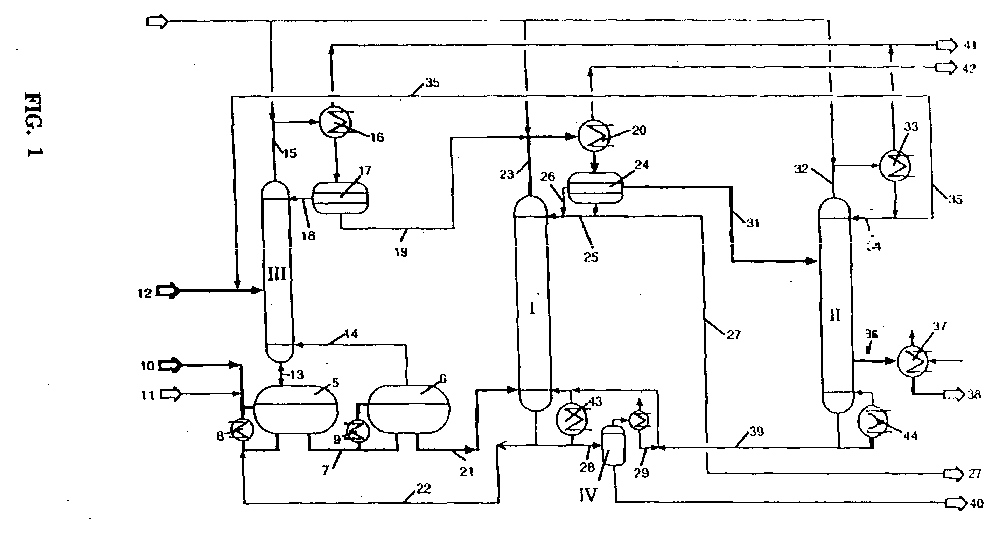

Weitere Einzelheiten und Vorteile der Erfindung können den im folgenden anhand der Zeichnung beschriebenen Ausführungsbeispielen entnommen werden, wobei die Zeichnung
in Fig. 1 eine schematische Darstellung einer Anlage zur Herstellung von n-Butylacrylat
zeigt.

Die Rektifikationskolonnen(-einheiten) sind mit römischen Bezugszeichen versehen. Der Übersichtlichkeit wegen sind außerdem die allgemein mit römischen Ziffern versehenen Produktbezeichnungen in den speziellen Ausführungsbeispielen noch präzisiert.

Die in der Fig.1 dargestellte Anlage zur Durchführung des erfindungsgemäßen Verfahrens für die Herstellung von n-Butylacrylat hat drei Rektifikationskolonnen I, II, III und eine Destillationseinheit IV; Butanol steht für n-Butanol. Sie ist außerdem mit zwei Veresterungsreaktoren 5 und 6 versehen, die über eine Leitung 7 hintereinander geschaltet sind und so eine Reaktionskaskade bilden. An die Reaktoren 5 und 6 sind Umlaufverdampfer 8 und 9 angeschlossen. Über die Leitung 10 wurden 4 mol/h Acrylsäure dem ersten Reaktor 5 und über die auf den ersten Reaktor 5 aufgesetzte Kolonne III 4 mol/h Butanol zugeführt, das in diese Kolonne III durch die Leitung 12 eingeleitet wurde. In den ersten Reaktor 5 wurde außerdem als Katalysator wäßrige p-Toluolsulfonsäure über die Leitung 11 eingeführt in einer Menge von 1,5 Gew.-% bezogen auf die eingesetzten Edukte. Die Umsetzung im ersten Reaktor 5 wurde bei einer Temperatur von 100°C, im nachgeschalteten zweiten Reaktor 6 bei einer Temperatur von 105°C bei einem Systemdruck von 380 mbar und einer Verweilzeit von ca. 3 h in der Reaktionszone durchgeführt.

Die aus den Reaktoren 5 und 6 aufsteigendem Dämpfe wurden durch Leitungen 13 und 14 in eine Glockenbodenkolonne III als erste Rektifiziereinheit eingeleitet und dort rektifiziert. Das Kopfprodukt dieser Kolonne III war frei von Acrylsäure. Es wurde in einem Oberflächenkondensator 16 kondensiert und in einen Abscheider 17 geführt. Dort trennte sich eine organische Phase, die 70 Gew.% Butanol, 12 Gew.-% Butylacrylat, ≤ 13 Gew.-% Wasser, 4 Gew.-% Butylacetat und 2000 ppm Dibutylether enthielt. Sie wurde vollständig durch die Leitung 18 als Rücklauf in die Kolonne III zurückgeführt. Die bei der Trennung entstandene wäßrige Phase, die noch 6 Gew.% Butanol, 300 ppm Butylacrylat, 750 ppm Butylacetat enthielt, wurde zur Verschiebung des Reaktionsgleichgewichts vollständig abgetrennt und über die Leitung 19 dem Dekanter 24 der nachfolgenden Rektifikationskolonne I zugeführt.

Der aus dem zweiten Reaktor 6 flüssig ablaufende Rohester wurde über Leitung 21 der Rektifikationskolonne I zugeführt. Er enthielt neben 0,2 Gew.-% Wasser und maximal 20 ppm Dibutylether das gewünschte Produkt n-Butylacrylat zu 78 Gew.-%, die nicht umgesetzten Edukte Butanol und Acrylsäure zu je ca. 4 Gew.-%, sowie ca. 5 Gew.-% Katalysator. Der Rest waren schwersiedende Nebenprodukte, insbesondere Oxyesterverbindungen.

Acrylsäure und Schwersieder wurden in der bei Umgebungsdruck betriebenen, mit 25 Siebböden ausgestatteten Rektifikationskolonne I mit einem Teil des Produktes und des Alkohols als Sumpfprodukt (Prod. II) abgetrennt. Das Sumpfprodukt (Prod. II) enthielt 20 Gew.-% Acrylsäure, 45 Gew.-% Butylacrylat, 3 Gew.-% Butanol, 8 Gew.-% Wasser. Eine Teilmenge von ca. 45 Gew.-% der durch die Leitung 21 zugeführten Zulaufmenge wurde über die Leitung 22 in den ersten Reaktionsbereich zurückgeführt.

Der Großteil der Schwersieder (bis zu 80 % der zugeführten Menge) wurde dabei im Sumpf der Rektifikationskolonne I in Edukte und Produkte gespalten. Dabei entstanden aufgrund des hohen Acrylsäure- und Wassergehaltes des Sumpfproduktes nur unwesentliche Mengen an leichtsiedenden Nebenprodukten (≤ 200 ppm Dibutylether). Diese Nebenprodukte wurden mit dem Hauptproduktstrom als leichtsiedendes MinimumHeteroazeotrop über Kopf der Kolonne I abgetrennt und über Leitung 23 dem Kondensator 20 zugeführt. Sowohl die Flüssigkeit in der Kolonne, als auch das Kopfprodukt zerfielen dabei in eine wäßrige und eine organische Phase. Zur Aufrechterhaltung des Heteroazeotropes in der Kolonne I wurde diese aus dem Kopfkondensator 24 mit wäßriger Phase durch Leitung 25 und mit organischer Phase durch Leitung 26 als Rücklauf beaufschlagt. Die wäßrige Phase hatte dabei ≤ 3 Gew.-% organische Bestandteile, vorwiegend Butanol. Die organischer Phase hatte 75 bis 85 Gew.-% Butylacrylat, 14 bis 20 Gew.-% Butanol, 2 bis 3 Gew.-% Wasser, 1500 ppm Butylacetat. Das dem Reaktionsumsatz entsprechende Überschußwasser wurde durch Leitung 27 ausgeschleust.

Auf die Zulaufmenge an Edukten in die Veresterung bezogen, wurden 5 Gew.-% des Sumpfproduktes (Prod. II) über eine Leitung 28 ausgeschleust und einem Rührkessel IV zugeführt. Dort wurde das Produkt bei Umgebungsdruck und 180°C bis zum deutlichen Viskositätsanstieg diskontinuierlich eingeengt. Zunächst wurden die darin noch enthaltenen Edukte Butanol und Acrylsäure neben dem Produkt Butylacrylat abdestilliert. Der Destillatanteil, bezogen auf die Einsatzmenge, betrug bis ca. 65 Gew.-%. Bei der anschließenden Spaltung der Schwersieder, der Sumpfaustrag wurde dabei bis auf ca. 15 % seiner ursprünglichen Masse eingeengt, wurden erst gegen Ende leichtsiedende Nebenprodukte wie Butene und Dibutylether in geringem Umfang gebildet. Der niedergeschlagene Brüden aus der Spaltung im Rührkessel IV bestand im wesentlichen aus Acrylsäure, Butylacrylat, Butanol und Wasser. Er wurde unmittelbar in den Sumpf der Kolonne I für die Schwersiederabtrennung zurückgeschleust. Eine weitere Rektifikation erfolgte nicht.

Das schwersieder- und acrylsäurefreie organische Kopfprodukt (Prod. I) der Azeotropdestillation in der Kolonne I wurde über die Leitung 31 einer mit 25 Böden versehenen Destillationskolonne II zugeführt und dort rektifiziert. Butanol, Restwasser und evtl. enthaltene Leichtsieder wurden dabei als Kopfprodukt über Leitung 32 abgezogen (Prod. V). Es enthielt 65 bis 70 Gew.-% Butanol, 20 bis 30 Gew.-% Butylacrylat, 8 bis 10 Gew.-% Wasser, ≤ 500 ppm Dibutylether, ≤ 4000 ppm Butylacetat. Dieses Kopfprodukt (Prod. V) wurde in einem Kondensator 33 kondensiert, und eine Teilmenge wurde durch Leitung 34 als Rücklauf auf den Kopf der Rektifikationskolonne II zurückgeführt. Die Hauptmenge wurde durch Leitung 35 zusammen mit dem durch Leitung 12 zugeführten Frischalkohol der Veresterung über die erste Kolonne 1 zugeführt. Das Butylacrylat wurde im Sumpf dieser Kolonne II aufkonzentriert und zur Einhaltung der gewünschten Farbzahl sowie zur Abtrennung des Prozeßstabilisators als dampfförmiger Seitenaustrag durch Leitung 36 abgezogen, im Kondensator 37 kondensiert und durch Leitung 38 abgeführt. Das Reinprodukt enthielt ≤ 50 ppm Butanol, ≤ 50 ppm Dibutylether, ≤ 150 ppm Wasser, ≤ 50 ppm Acrylsäure.

Ein kleiner Sumpfabzugsstrom (Prod. VI), in einer Menge ≤ 2 Gew.-% des Kolonnenzulaufes, wurde über eine Leitung 39 zum Sumpf der Schwersiederabtrennung in die Kolonne I geleitet.

Durch die Leitung 40 wurde aus dem Rührbehälter IV der Rückstand ausgetragen. Leitung 41 verband die Kolonnen III und II mit einer Vakuumpumpe. Durch die Leitung 42 wurde Abluft von der Kolonne I abgeführt. Der Sumpf der Kolonnen I und II wurde über Umlaufverdampfer 43 bzw. 44 beheizt.

Der Reinester wies eine Reinheit von ≥ 99.9 % auf, die Ausbeute bezogen auf Acrylsäure und Butanol betrug jeweils 98 % der Theorie.

In einem weiteren Versuch wurde der zweite Veresterungsreaktor 6 außer Betrieb gesetzt und der Rohester aus dem ersten Reaktor 5 über die Leitung 7 direkt in den Sumpf der Kolonne I eingeleitet. Die Umsetzung wurde bei 105°C durchgeführt. Bei gleichbleibenden Feedströmen, und damit gegenüber der Variante mit zwei Reaktoren verringerter Verweilzeit, konnte bei sonst identischen Verfahrensparametern ein Rohester mit 71 Gew.-% des gewünschten Produktes n-Butylacrylat, 0,4 Gew.-% Wasser, maximal 20 ppm Dibutylether, einem Eduktgehalt (Butanol und Acrylsäure) von je ca. 7 Gew.-% sowie bis zu 5 Gew.-% Katalysator erhalten werden. Der Rest waren schwersiedende Nebenprodukte, insbesondere Oxiesterverbindungen.

Der so erzeugte Rohester wurde analog zum ersten Versuch, bei identischen Verfahrensparametern im Aufarbeitungsteil, zu 99,9 %-igem Reinprodukt bei einer auf die Edukte bezogener Gesamtausbeute von 98% aufgereinigt.

## Patentansprüche

1. Verfahren zur kontinuierlichen Herstellung von Alkylestern der (Meth)acrylsäure durch Umsetzung von (Meth)acrylsäure mit *4 oder 5* C-Atome aufweisenden Alkanolen in homogener, flüssiger, an Lösungsmittel freier Phase bei erhöhter Temperatur und in Gegenwart eines sauren Veresterungskatalysators, bei dem man die (Meth)acrylsäure, das Alkanol und den Katalysator einer Reaktionszone zuführt *(5, 6)*, während einer Verweilzeit das gebildete Wasser als Bestandteil eines Alkanol umfassenden Gemisches in einer der Reaktionszone *(5, 6)* aufgesetzten Rektifikationseinheit *(III)* rektifikativ abtrennt, das dabei anfallende Destillat in eine Alkanol enthaltende organische und in eine Wasser enthaltende wäßrige Phase auftrennt, die organische Phase in die Rektifikationseinheit *(III)* zurückführt, das Reaktionsgemisch aus der Reaktionszone *(5, 6)* austrägt und in eine weitere Rektifikationseinheiten *(I, II)* umfassende destillative Trennzone leitet und in dieser den gebildeten Alkylester der (Meth)acrylsäure abtrennt, **dadurch gekennzeichnet**, daß
a) (Meth)acrylsäure und Alkanol im Molverhältnis 1:0,75 bis 1:2 umgesetzt werden,
b) die in der Rektifikationseinheit (III) anfallende organische Phase vollständig oder *mit Ausnahme eines Teilstroms, der ausgeschleust wird,* in die Rektifikationseinheit *(III)* zurückgeführt wird,
c) die in der Rektifikationseinheit (III) anfallende wäßrige Phase ausgeschleust wird,
d) das aus der Reaktionszone ausgetragene Reaktionsgemisch unter Zusatz von Wasser einer weiteren Rektifikationseinheit (I) zugeführt und in dieser in ein den Katalysator und die verbliebene (Meth)acrylsäure enthaltendes Produkt (II) und in ein den Alkylester der (Meth)acrylsäure, verbliebenes Alkanol und Wasser enthaltendes Produkt (I) aufgetrennt wird,
e) das in der Rektifikationseinheit (I) anfallende Produkt (II) *mit Ausnahme eines Teilstroms, der ausgeschleust wird* in die Reaktionszone zurückgeführt wird,
f) das Produkt (I) der Rektifikationseinheit (I) in eine den Alkylester der (Meth)acrylsäure enthaltende organische Phase und in eine wäßrige Phase aufgetrennt und
g) die in der Rektifikationseinheit (I) anfallende organische Phase einer weiteren Rektifikationseinheit (II) zugeführt und in dieser der Alkylester der (Meth)acrylsäure vom verbliebenen Alkanol abgetrennt und das verbliebene Alkanol in die Reaktionszone zurückgeführt wird.

2. Verfahren nach Anspruch 1, **dadurch gekennzeichnet**, daß die Reaktionszone (5, 6) aus einer Kaskade von mindestens zwei hintereinander geschalteten Reaktionsbereichen besteht, die insbesondere 2 bis 4 voneinander räumlich getrennte Reaktionsbereiche aufweist, und der Austragsstrom eines Reaktionsbereichs einen Zulaufstrom eines nachfolgenden Reaktionsbereichs bildet, wobei insbesondere die Temperatur im ersten Reaktionsbereich 70 - 150°C und inn letzten Bereich 100 - 160°C beträgt.

3. Verfahren nach Anspruch 2, **dadurch gekennzeichnet**, daß der Druck in allen Reaktionsbereichen von 100 mbar bis Atmosphärendruck *beträgt und* in allen Reaktionsbereichen gleich ist, *und daß* die Gesamtverweilzeit der Reaktanden in den Reaktionsbereichen 0,25 bis 15 h beträgt.

4. Verfahren nach einem der Ansprüche 1 bis 3, **dadurch gekennzeichnet**, daß die aufsteigenden Dämpfe aus den Reaktionsbereichen *(5, 6)* einer Rektifikationseinheit (III) zugeführt werden, deren flüssiger Rücklauf nur in den ersten Reaktionsbereich *(5)* zurückgeführt wird.

5. Verfahren nach einem der Ansprüche 1 bis 4, **dadurch gekennzeichnet**, daß als Katalysator para-Toluolsulfonsäure und/oder eine andere organische Sulfonsäure wie Methansulfonsäure, Benzolsulfonsäure, Dodecylbenzolsulfonsäure und/oder Schwefelsäure eingesetzt werden, wobei der Gehalt an katalytisch wirksamer Säure in der Reaktionszone, bezogen auf das darin enthaltene Reaktionsgemisch, 0,1 bis 10 Gew.-% an para-Toluolsulfonsäure beträgt oder einer dazu äquimolaren Menge an einer anderen organischen Sulfonsäure und/oder Schwefelsäure, und der Gehalt an katalytisch wirksamer Säure im Sumpf der Rektifikationseinheit (I), bezogen auf das darin enthaltene Gemisch, zwischen 2,5 und 25 Gew.-% para-Toluolsulfonsäure beträgt und/oder einer dazu äquimolaren Menge an einer anderen organischen Sülfonsäure und/oder Schwefelsäure.

6. Verfahren nach einem der Ansprüche 1 bis 5, **dadurch gekennzeichnet**, daß sowohl die (Meth)acrylsäure als auch der Katalysator der Reaktionszone *(5, 6)* direkt zugeführt wird, das zu veresternde Alkanol, insbesondere n-Butanol, der Reaktionszone über die Rektifikationseinheit (III) zugeführt wird, die Rektifikationseinheit (III) aus einer Rektifikationskolonne besteht, die Reaktionsbereiche *(5, 6)* aus Reaktoren mit Umlaufverdampfern bestehen, und die am Kopf der Rektifikationseinheit (III) anfallende wäßrige Phase vollständig ausgeschleust wird.

7. Verfahren nach Anspruch 6, **dadurch gekennzeichnet**, daß bei Einsatz von n-Butanol das Produkt (I) in eine den n-Butylester der (Meth)acrylsäure und n-Butanol enthaltende organische Phase und in eine wäßrige Phase aufgetrennt wird, ein Teil der wäßrigen Phase in die Rektifikationseinheit (I) zurückgeführt wird, in der Rektifikationseinheit (I) eine flüssige wäßrige und eine flüssige organische Phase vorhanden sind, die Rektifikationseinheit (I) eine Rektifikationskolonne (I) ist, das aus der Reaktionszone ausgetragene Produktgemisch dem unteren Teil der Rektifikationskolonne (I) zugeführt wird und der Wasserzusatz im oberen Teil der Rektifikationskolonne (I) erfolgt, und wobei ein Teil der den n-Butylester der (Meth)acrylsäure und n-Butanol enthaltenden anfallenden organischen Phase in den oberen Teil der Rektifikationskolonne (I) zurückgeführt wird.

8. Verfahren nach Anspruch 6 oder 7, **dadurch gekennzeichnet**, daß das in der Rektifikationseinheit (I) anfallende, den Katalysator und die verbliebene (Meth)acrylsäure enthaltende Produkt (II) im wesentlichen vollständig in die Reaktionszone, in den ersten Reaktionsbereich, entweder direkt und/oder über die Rektifikationseinheit (III), zurückgeführt wird, wobei ein Teil des in der Rektifikationseinheit (I) anfallenden Produktes (II) ausgetragen und einer Destillationseinheit (IV) zugeführt und in dieser in ein n-Butanol, (Meth)acrylsäure und n-Butylester der (Meth)acrylsäure enthaltendes Produkt (III) und ein den Katalysator sowie höher als den n-Butylester der (Meth)acrylsäure siedende Komponenten enthaltendes Produkt (IV) aufgetrennt wird, und das Produkt (III) in die Rektifikationseinheit (I) und/oder in die Reaktionszone zurückgeführt wird.

9. Verfahren nach einem der Ansprüche 6 bis 8, **dadurch gekennzeichnet**, daß die organische Phase des Produkts (I) einer Rektifikationseinheit (II) zugeführt und in dieser aufgetrennt wird in ein verbliebenes n-Butanol und leichter als n-Butyl(meth)acrylat siedende Komponenten enthaltendes Produkt (V), n-Butyl(meth)acrylat und ein schwerer als n-Butyl(meth)acrylat siedendes Produkt (VI), wobei das Produkt (V) in die Reaktionszone zurückgeführt wird, vorzugsweise über die Rektifikationseinheit (III), das Produkt (VI) in die Rektifikationseinheit (I) zurückgeführt wird, die Rektifikationseinheit (II) eine Rektifikationskolonne (II) ist, und wobei das Produkt (V) im oberen Teil der Rektifikationskolonne (II), das Produkt (VI) dem Sumpf der Rektifikationskolonne (II) und das n-Butyl(meth)acrylat als dampfförmiger Seitenabzug im unteren Teil der Rektifikationskolonne (II) aufsteigend abgetrennt werden.

10. Vorrichtung zur Durchführung des Verfahrens zur kontinuierlichen Herstellung von Alkylestern der (Meth)acrylsäure nach einem der Ansprüche 1 bis 9 mit einem ersten Reaktor (5), der mit Zuführungsleitungen (10) für einen der Reaktanden und einer Zuführungsleitung (11) für den Katalysator versehen ist, und einem zweiten Reaktor (6), die über einen Überlauf (7) miteinander verbunden sind und deren beide Kopfteile über Leitungen (13,14) mit dem unteren Teil einer Rektifikationseinheit (III) verbunden sind, deren Kopf über einen Kondensator (16) mit einem Abscheider (17) in Verbindung steht, der eine Abführungsleitung (19) für das Reaktionswasser zu einem Abscheider (24) aufweist, und wobei der Abscheider (24) über einen Kondensator (20) mit dem Kopf einer Rektifikationskolonne (I) verbunden ist und der zweite Reaktor (6) über eine Leitung (21) mit der Rektifikationskolonne (I) verbunden ist, deren Sumpf über eine Leitung (22) mit dem ersten Reaktor (5) verbunden ist sowie über eine Leitung (28) mit einer Destillationseinheit (IV).

## Claims

1. A process for the continuous preparation of alkyl esters of (meth)acrylic acid by reacting (meth)acrylic acid and alkanols having 4 or 5 carbon atoms in a homogeneous, liquid, solvent-free phase at elevated temperature and in the presence of an acid esterification catalyst, in which the (meth)acrylic acid, the alkanol and the catalyst are fed to a reaction zone (5, 6), the water formed is removed by rectification during a residence time as constituent of a mixture comprising alkanol in a rectification unit (III) superposed on the reaction zone (5, 6), the distillate thus obtained is separated into an organic phase comprising alkanol and an aqueous phase comprising water, the organic phase is returned to the rectification unit (III), the reaction mixture is discharged from the reaction zone (5, 6) and conveyed into a distillative separation zone comprising further rectification units (I, II) and in the latter the alkyl (meth)acrylate formed is separated off, wherein
a) (meth)acrylic acid and alkanol are reacted in a molar ratio of from 1:0.75 to 1:2,
b) the organic phase formed in the rectification unit (III) is completely, or with the exception of a substream which is bled off, returned to the rectification unit (III),
c) the aqueous phase formed in the rectification unit (III) is removed from the system,
d) the reaction mixture discharged from the reaction zone is, with addition of water, fed to a rectification unit (I) and in this is separated into a product (II) comprising the catalyst and the remaining (meth)acrylic acid and a product (I) comprising the alkyl ester of (meth)acrylic acid, remaining alkanol and water,
e) the product (II) formed in the rectification unit (I) is with the exception of a substream which is bled off, returned to the reaction zone,
f) the product (I) from the rectification unit (I) is separated into an organic phase comprising the alkyl ester of (meth)acrylic acid and an aqueous phase and
g) the organic phase formed in the rectification unit (I) is fed to a further rectification unit (II) and in this the alkyl ester of (meth)acrylic acid is separated from the remaining alkanol and the remaining alkanol is returned to the reaction zone.

2. A process as claimed in claim 1, wherein the reaction zone (5, 6) comprises a cascade of at least two reaction regions connected in series which has, in particular, from 2 to 4 reaction regions separated from one another in space, and the discharge stream of one reaction region forms a feed stream to a downstream reaction region, the temperature in the first reaction region being 70-150°C, and in the last region being 100-160°C.

3. A process as claimed in claim 2, wherein the pressure in all reaction regions is from 100 mbar to atmospheric pressure and is the same in all reaction regions, and wherein the total residence time of the reactants in the reaction regions is from 0.25 to 15 hours.

4. A process as claimed in any of claims 1 to 3, wherein the rising vapors from the reaction regions (5, 6) are fed to a rectification unit (III) whose liquid runback is returned only to the first reaction region (5).

5. A process as claimed in any of claims 1 to 4, wherein the catalyst used comprises para-toluenesulfonic acid and/or another organic sulfonic acid such as methanesulfonic acid, benzenesulfonic acid, dodecylbenzenesulfonic acid and/or sulfuric acid, the content of catalytically active acid in the reaction zone, based on the reaction mixture present therein, being from 0.1 to 10% by weight of para-toluenesulfonic acid or an amount equimolar thereto of another organic sulfonic acid and/or sulfuric acid, and the content of catalytically active acid in the liquid phase of the rectification unit (I), based on the mixture present therein, being from 2.5 to 25% by weight of paratoluenesulfonic acid and/or an amount equimolar thereto of another organic sulfonic acid and/or sulfuric acid.

6. A process as claimed in any of claims 1 to 5, wherein both the (meth)acrylic acid and the catalyst are fed directly to the reaction zone (5, 6) the alkanol to be esterified, particularly n-butanol, is fed to the reaction zone via the rectification unit (III), the rectification unit (III) is a rectification column, the reaction regions (5, 6) comprise reactors having convection vaporizers, and the aqueous phase obtained at the top of the rectification unit (III) is completely discharged.

7. A process as claimed in claim 6, wherein, when n-butanol is used, the product (I) is separated into an organic phase comprising the n-butyl ester of (meth)acrylic acid and n-butanol and an aqueous phase, part of the aqueous phase is returned to the rectification unit (I), a liquid aqueous phase and a liquid organic phase are present in the rectification unit (I), the rectification unit I is a rectification column (I), the product mixture discharged from the reaction zone is fed to the lower part of the rectification column (I) and the water addition occurs in the upper part of the rectification column (I), and where part of the resulting organic phase comprising the n-butyl ester of (meth)acrylic acid and n-butanol is returned to the upper part of the rectification column (I).

8. A process as claimed in claim 6 or 7, wherein the product (II) formed in the rectification unit (I) and comprising the catalyst and the remaining (meth)acrylic acid is returned essentially completely to the reaction zone, to the first reaction region, either directly and/or via the rectification unit (III), part of the product (II) formed in the rectification unit (I) being discharged and fed to a distillation unit (IV) and in this being separated into a product (III) comprising n-butanol, (meth)acrylic acid and the n-butyl ester of (meth)acrylic acid and a product (IV) comprising the catalyst and components having higher boiling points than the n-butyl ester of (meth)acrylic acid, and the product (III) being returned to the rectification unit (I) and/or the reaction zone.

9. A process as claimed in any of claims 6 to 8, wherein the organic phase of the product (I) is fed to a rectification unit (II) and in this is separated into a product (V) comprising remaining n-butanol and components having lower boiling points than n-butyl (meth)acrylate, n-butyl (meth)acrylate and a product (VI) having a boiling point higher than n-butyl (meth)acrylate, the product (V) being returned to the reaction zone, preferably via the rectification unit (III), the product (VI) being returned to the rectification unit (I), the rectification unit (II) being a rectification column (II), and the product (V) being taken off in the upper part of the rectification column (II), the product (VI) being taken off from the bottom of the rectification column (II) and the n-butyl (meth)acrylate being taken off in vapor form while rising as a lateral branch stream in the lower part of the rectification column (II).

10. An apparatus for carrying out the process for the continuous preparation of alkyl esters of (meth)acrylic acid as claimed in any of claims 1 to 9 comprising a first reactor (5) which is provided with a feed line (10) for one of the reactants and a feed line (11) for the catalyst, and a second reactor (6) which are connected to one another via an overflow (7) and whose two top parts are connected via lines (13, 14) to the lower part of a rectification unit (III), the top of which is connected via a condenser (16) to a separator (17) having a discharge line (19) for the water of reaction to a separator (24), and where the separator (24) is connected via a condenser (20) to the top of a rectification column (I) and the second reactor (6) is connected via a line (21) to the rectification column (I) whose bottom is connected via a line (22) to the first reactor (5) and via a line (28) to a distillation unit (IV).

## Revendications

1. Procédé de préparation continue d'esters alkyliques de l'acide (méth)acrylique, par réaction d'acide (méth)acrylique avec des alcanols ayant 4 ou 5 atomes de carbone, dans une phase homogène, liquide, exempte de solvant, à haute température et en présence d'un catalyseur de transestérification, dans lequel on envoie l'acide (méth)acrylique, l'alcanol et le catalyseur dans une zone de réaction (5, 6) ; pendant un temps de séjour, on sépare par rectification, dans une unité de rectification (III) disposée au-dessus de la zone de réaction (5, 6), l'eau qui se forme en tant que constituant d'un mélange contenant un alcanol ; on sépare le distillat qui se forme à cette occasion en une phase organique contenant un alcanol et une phase aqueuse contenant de l'eau ; on renvoie la phase organique dans l'unité de rectification (III) ; on extrait le mélange réactionnel de la zone de réaction (5, 6), et on l'envoie dans une zone de séparation par distillation, comportant d'autres unités de rectification (I, II), et on y sépare l'ester alkylique ainsi formé de l'acide (méth)acrylique, **caractérisé en ce que**
a) on fait réagir l'acide (méth)acrylique et l'alcanol selon un rapport en moles de 1:0,75 à 1:2,
b) on renvoie dans l'unité de rectification (III) la phase organique qui se forme dans l'unité de rectification (III), en totalité ou à l'exception d'un courant partiel soutiré,
c) on soutire la phase aqueuse qui se forme dans l'unité de rectification (III),
d) on envoie à une autre unité de rectification (I), avec addition d'eau, le mélange réactionnel sortant de la zone de réaction, et on y sépare ce mélange en un produit (II) contenant le catalyseur et l'acide (méth)acrylique résiduel, et un produit (I), contenant l'ester alkylique de l'acide (méth)acrylique, l'alcanol résiduel et de l'eau,
e) on renvoie dans la zone de rectification le produit (II) qui se forme dans l'unité de rectification (I), à l'exception d'un courant partiel qui est soutiré,
f) on sépare le produit (I) de l'unité de rectification (I) en une phase organique contenant l'ester alkylique de l'acide (méth)acrylique et en une phase aqueuse, et
g) on envoie la phase organique qui se forme dans l'unité de rectification (I) dans une autre unité de rectification (II), et, dans cette dernière, on sépare de l'alcanol résidu l'ester alkylique de l'acide (méth)acrylique, et on renvoie dans la zone de réaction l'alcanol résiduel.

2. Procédé selon la revendication 1, **caractérisé en ce que** la zone de réaction (5, 6) est constituée d'une cascade d'au moins deux domaines de réaction montés l'un derrière l'autre, qui en particulier comporte 2 à 4 domaines de réaction spatialement séparés les uns des autres, et le courant de sortie d'un domaine de réaction forme un courant de charge d'un domaine de réaction suivant, auquel cas en particulier la température dans le premier domaine de réaction est de 70 à 150°C et dans le dernier domaine de 100 à 160°C.

3. Procédé selon la revendication 2, **caractérisé en ce que** la pression dans tous les domaines de réaction est comprise entre 100 mbar et la pression atmosphérique et est identique dans tous les domaines de réaction, et que le temps total de séjour des réactifs dans les domaines de réaction est de 0,25 à 15 h.

4. Procédé selon l'une des revendications 1 à 3, **caractérisé en ce que** les vapeurs ascendantes provenant des domaines de réaction (5, 6) sont envoyées à une unité de rectification (III) dont le retour liquide n'est renvoyé que dans le premier domaine de réaction (5).

5. Procédé selon l'une des revendications 1 à 4, **caractérisé en ce qu**'on utilise en tant que catalyseur de l'acide paratoluènesulfonique et/ou un autre acide sulfonique organique, tel que l'acide méthanesulfonique, l'acide benzènesulfonique, l'acide dodécylbenzènesulfonique et/ou l'acide sulfurique, où la quantité de l'acide à effet catalytique dans la zone de réaction, rapportée au mélange réactionnel qui s'y trouve, est de 0,1 à 10 % en poids d'acide paratoluènesulfonique, ou une quantité équimolaire d'un autre acide sulfonique et/ou sulfurique organique, et la quantité d'acide à effet catalytique dans le fond de l'unité de rectification (II), rapportée au mélange qui s'y trouve, est comprise entre 2,5 et 25 % en poids d'acide paratoluènesulfonique et/ou une quantité équimolaire d'un autre acide sulfonique et/ou sulfurique organique.

6. Procédé selon l'une des revendications 1 à 5, **caractérisé en ce que** tant l'acide (méth)acrylique que le catalyseur sont directement envoyés à la zone de réaction (5, 6) ; l'alcanol à estérifier, en particulier le n-butanol, est envoyé à la zone de réaction par l'intermédiaire de l'unité de rectification (III) ; l'unité de rectification (III) est constituée d'une colonne de rectification ; les domaines de réaction (5, 6) sont constitués de réacteurs comportant des évaporateurs à recirculation ; et la phase aqueuse qui se forme en tête de l'unité de rectification (III) est entièrement soutirée.

7. Procédé selon la revendication 6, **caractérisé en ce que**, quand on utilise du n-butanol, le produit (I) est séparé en une phase organique contenant l'ester n-butylique de l'acide (méth)acrylique et du n-butanol et en une phase aqueuse ; une partie de la phase aqueuse est renvoyée dans l'unité de rectification (I) ; une phase aqueuse liquide et une phase organique liquide sont présentes dans l'unité de rectification (I) ; l'unité de rectification (I) est une colonne de rectification (I) ; le mélange de produits extrait de la colonne de réaction est envoyé à la partie inférieure de la colonne de rectification (I); et l'addition d'eau s'effectue dans la partie supérieure de la colonne de rectification (I); et une partie de la phase organique obtenue, qui contient l'ester n-butylique de l'acide (méth)acrylique et du n-butanol, est renvoyée dans la partie supérieure de la colonne de rectification (I).

8. Procédé selon la revendication 6 ou 7, **caractérisé en ce que** le produit (II), que l'on obtient dans l'unité de rectification (I) et qui contient le catalyseur et l'acide (méth)acrylique résiduel, est pour ainsi dire complètement renvoyé dans la zone de réaction, dans le premier domaine de réaction, directement et/ou par l'intermédiaire de l'unité de rectification (III) ; auquel cas une partie du produit (II) que l'on obtient dans l'unité de rectification (I) est évacuée et est envoyée à une unité de distillation (IV) et y est séparée en un produit (III) contenant du n-butanol, de l'acide (méth)acrylique et l'ester n-butylique de l'acide (méth)acrylique et en un produit (IV) contenant le catalyseur ainsi que des composants dont le point d'ébullition est plus élevé que celui de l'ester n-butylique de l'acide (méth)acrylique ; et le produit (III) est renvoyé dans l'unité de rectification (I) et/ou dans la zone de réaction.

9. Procédé selon l'une des revendications 6 à 8, **caractérisé en ce que** la phase organique du produit (I) est envoyée à une unité de rectification (II) et y est séparée en un produit (V) contenant le n-butanol résiduel et les composants ayant un point d'ébullition plus bas que celui du (méth)acrylate de n-butyle, en (méth)acrylate de n-butyle et en un produit (VI) ayant un point d'ébullition plus élevé que le (méth)acrylate de n-butyle ; où le produit (V) est renvoyé dans la zone de réaction, de préférence par l'intermédiaire de l'unité de rectification (III) ; le produit (VI) est renvoyé dans l'unité de rectification (I) ; l'unité de rectification (II) est une colonne de rectification (II) ; et on sépare, par séparation ascendante, le produit (V) dans la partie supérieure de la colonne de rectification (II), le produit (VI) dans le fond de la colonne de rectification (II) et le (méth)acrylate de n-butyle, sous forme d'un soutirage latéral en phase vapeur, dans la partie inférieure de la colonne de rectification (II).

10. Appareillage pour mettre en oeuvre le procédé de préparation continue d'esters alkyliques de l'acide (méth)acrylique selon l'une des revendications 1 à 9, comportant un premier réacteur (5), qui est pourvu de conduites d'amenée (10) pour l'un des réactifs et d'une conduite d'amenée (11) pour le catalyseur, et un deuxième réacteur (6), les deux réacteurs étant reliés l'un à l'autre par un trop-plein (7), et leurs deux parties de tête étant reliées par l'intermédiaire de conduites (13, 14) à la partie inférieure d'une unité de rectification (III) dont la tête est reliée par l'intermédiaire d'un condenseur (16) à un séparateur (17), qui comporte une conduite d'évacuation (19), destinée à l'eau de réaction et allant vers un séparateur (24), le séparateur (24) étant par l'intermédiaire d'un condenseur (20) relié avec la tête d'une colonne de rectification (I), et le deuxième réacteur (6) étant par l'intermédiaire d'une conduite (21) relié à la colonne de rectification (I), dont le fond est par l'intermédiaire d'une conduite (22) relié au premier réacteur (5), et par l'intermédiaire d'une conduite (28) à une unité de distillation (IV).
